# EUROPEAN PATENT APPLICATION

(11) **EP 4 850 646 A1**
(43) Date of publication of application: **09.12.2026**
(21) Application number: 26182186.2
(22) Date of filing: 01.06.2026
(51) Int. Cl.: A61F 13/02, A61F 13/00

(54) **PROCESS AND LINE FOR THE PRODUCTION OF MEDICAL PATCHES FOR THE TREATMENT OF PAIN BY MEANS OF HEAT**

(30) Priority: 03.06.2025 IT 202500012865; 03.06.2025 IT 202500012868
(71) Applicant: Eurosirel S.p.A., 20047 Cusago (MI) (IT)
(72) Inventor: Leonelli, Ernesto, 20047 Cusago (MI) (IT)
(74) Representative: Perani & Partners S.p.A.

(57) **Abstract**

A process for the production of medical patches (1) for treating pain by means of heat, comprising the steps of:
A) continuously feeding a laminate (2) having:
- a first layer (3) made of polymeric material,
- a second layer (4), applied onto the first layer (3) and made of adhesive material, in which is dispersed a chemical compound (4a) configured to reflect at least part of the far infrared radiation (FIR) emitted by the body of the user of the medical patch (1),
- a protective layer (6) removably applied to the second layer (4) on the opposite side with respect to the first layer (3),

B) removing the protective layer (6) from the laminate (2) so as to progressively uncover the second layer (4),
C) applying a removable film (7) to the laminate (2) so as to progressively cover the second layer (4),
D) repeatedly die-cutting the laminate (2) so as to obtain a plurality of patches (1) arranged in succession on the film (7),
E) cutting the film (7) so as to separate the patches (1),
F) enclosing each patch (1) in a respective casing (8).

The process is characterized in that step C) of applying the film (7), comprises the substeps of:
C1) continuously feeding the film (7),
C2) cutting the fed film (7) to divide it into two or more strips (70),
C3) applying to the second layer (4) the strips (70) obtained in step C2) side by side with one another.

## Description

### Technical Field

The present invention relates to a process and a line for the production of medical patches intended for the treatment of pain - associated, for example, with inflammatory states, circulatory pathologies or muscular disorders -, through the application of heat.

### State of the Art

In the field of medical devices, there is a felt need to obtain medical patches for the treatment of pain that do not contain drugs or active ingredients that are released and absorbed by the user during treatment.

To this end, various medical devices in the form of a patch have been developed, configured to treat pain, in particular an inflammation in progress in the user, by means of heat. It has indeed been proven that the use of heat represents a valid method for treating inflammation, an alternative to traditional medical devices which, by carrying out a therapeutic action, release substances.

The category of medical devices for the treatment of pain by means of heat includes the so-called "FIR patches", i.e., patches that use the user's body as a source of heat for the treatment, in particular the electromagnetic waves belonging to the far infrared emitted by the user.

In detail, FIR patches are configured to intercept the (thermal) energy emitted by the user and reflect it or re-direct it towards the region of the user's body affected by the pain. The thermal energy reintroduced into the user's tissue acts on the organic tissues involved in inflammatory processes and in states of oxidative stress.

It has been found that traditional production processes for patches have some limitations in their use for the production of FIR patches.

Traditional methods of protecting the patches provide for repeatedly die-cutting a laminate comprising a support layer of non-woven fabric, an adhesive layer, and a removable protective film divided into two centrally overlapping strips. The latter, downstream of the die-cutting, form the protective flaps of the patch that the user removes before application.

In the case of FIR patches, the adhesive layer is typically constituted by a polymeric matrix loaded with a chemical compound suitable for reflecting the far infrared radiation emitted by the user's body. During the production process, the presence of overlaps between the strips of the protective film means that, in the die-cutting step, the pressure exerted induces localized deformations in the central region of the adhesive layer in correspondence with the overlapping portion of the protective flaps. These deformations create variations in the thickness of the adhesive layer loaded with the reflecting chemical compound and, therefore, alter the reflecting properties of the patch, partially compromising its functional effectiveness.

In addition, again due to the presence of overlaps between the strips of the protective film, the pressure applied during die-cutting is concentrated in the central region of the non-woven fabric support layer of the patch, generating localized deformations that give rise to structurally weakened areas, more subject to breakage and to the formation of folds. This can lead to difficulties in application or breakage of the patch, but also to the appearance of visible signs on the external surface of the product which mar its aesthetic appearance.

Therefore, in light of the above-mentioned critical issues, the need for an improved process and production line for medical patches for the treatment of pain by means of heat is evident.

### Object of the invention

In this context, the technical task underlying the present invention is to propose a process and a line for the production of patches for the treatment of pain by means of heat that overcomes the drawbacks of the known art mentioned above.

In particular, an object of the present invention is to provide a process and a line for the production of medical patches for the treatment of pain by means of heat that allows their structural integrity to be preserved so as to guarantee their optimal performance in terms of reflection of the radiation emitted by the user's body, as well as ease of application.

It is also an object of the present invention to provide a process and a line for the production of patches for the treatment of pain by means of heat capable of preventing the formation of visible signs on the outer layer of the patch.

### SUMMARY OF THE INVENTION

In accordance with the present invention, the indicated technical task and the specified objects are achieved by a process and a line for the production of patches for the treatment of pain by means of heat in accordance with one or more of the claims reported below.

In particular, the present invention, upstream of the die-cutting step, proposes to apply a removable film divided into two or more side-by-side strips on the adhesive material layer loaded with the compound suitable for reflecting at least part of the radiation emitted by the user's body.

This process characteristic is also reflected in the line according to the present invention which, upstream of the die-cutting station, has a replacement station configured to replace a protective layer of the laminate with a removable film divided into two or more side-by-side strips.

Differently from what happens in the known processes and lines, this allows to avoid the concentration of the pressure exerted by the die-cutting in specific regions of the patch and, therefore, the inconvenient formation of localized deformations of its layers.

It is therefore evident that the process and the line in accordance with the present invention, in addition to preventing the formation of visual defects on a first non-woven fabric layer of the patch (outer layer), is able to guarantee the uniformity of thickness of the adhesive layer loaded with the reflecting chemical compound, thus guaranteeing the optimal functional effectiveness of the patch as well as its easy application.

### LIST OF FIGURES

Further characteristics and advantages of the present invention will appear more clearly from the indicative, and therefore non-limiting, description of a preferred but not exclusive embodiment of a process and a line for the production of patches for the treatment of pain by means of heat, as illustrated in the accompanying drawings in which:

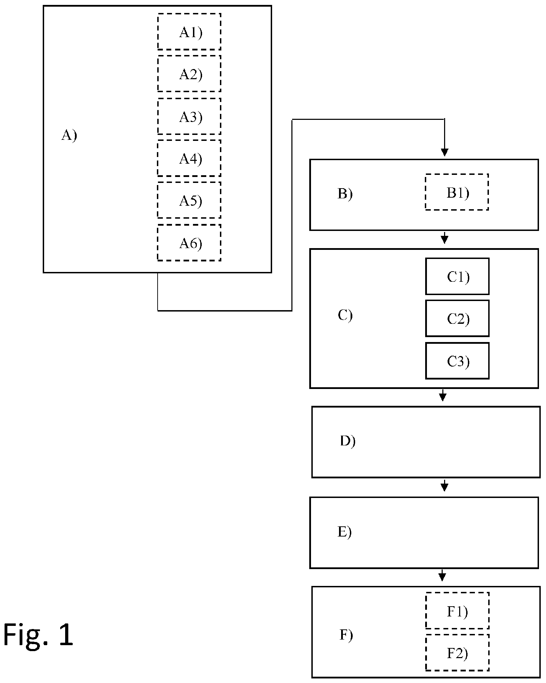

- Figure 1 shows a block diagram of a process for the production of patches for the treatment of pain according to the present invention in which the dashed blocks refer to optional steps of the production process,
- Figure 2 shows a schematic representation of a first embodiment of a line for the production of patches for the treatment of pain by means of heat adapted to implement the method schematized in figure 1,
- Figure 3 shows a schematic representation of a second embodiment of a line for the production of patches for the treatment of pain by means of heat adapted to implement the method schematized in figure 1,
- Figure 4 shows a schematic representation of a possible part of the line for the production of patches for the treatment of pain arranged upstream of the part shown in figures 2 and 3,
- Figure 5 shows a schematic representation of a third embodiment of a line for the production of patches for the treatment of pain by means of heat adapted to implement the method schematized in figure 1 with also at least part of the optional steps of the process,
- Figure 6 shows a perspective view of some details of a station of the line for the production of patches for the treatment of pain by means of heat according to the present invention,
- Figures 7 and 8 show some possible structures that the laminate with the applied removable film can assume immediately downstream of the station shown in figure 5,
- Figure 9 shows an embodiment of a patch for the treatment of pain by means of heat made through the process of figure 1.

### DETAILED DESCRIPTION

The present description has for object a process and a line for the production of medical patches 1 for the treatment of pain, in particular of inflammation, by means of heat.

It is emphasized that, both for the purposes of the present invention and as regards the known art, the term "patch" should not be understood in a restrictive sense but, on the contrary, this term is intended to mean a wearable support in various ways to deliver its treatment. Indeed, for the purposes of the present invention, a patch will not necessarily mean a device to be applied directly to the user's skin and, as in the known art, it can find application in the form of wearable supports in various ways, for example in the form of a band, insole or other. Naturally, also in such embodiments, suitable characteristics are provided to guarantee the correct positioning of the medical device.

It is well to specify that, according to one aspect, the medical patches 1 produced through the process and the line object of the present description do not contain drugs or active ingredients that are released and absorbed by the user during treatment.

In particular, preferably, the medical patches 1 produced through the process and the line object of the present description are FIR patches, i.e., patches that use the user's body as a source of heat for the treatment, in particular the electromagnetic waves belonging to the far infrared ("Far Infrared", or FIR hereinafter) emitted by the user. It is emphasized that FIR radiations with a wavelength between 4 and 20 µm are particularly effective for pain-relieving applications as they promote molecular phenomena of a vibrational "stretching" and "bending" type of the water molecule) and manifest a greater power of penetration into the human body, reaching depths of up to 6-7 cm with respect to the external surface of the epidermal layer. Further technical details about FIR patches and their operation are not further described here because they are known to the person skilled in the art. Indeed, there is extensive literature, both patent and non-patent, on these topics.

It is specified that, in use, the user does not perceive heat in the application region of the patch. That is to say that the patch does not cause a perceptible heating effect for the user. This is because the patch does not actively release heat but reflects the radiation emitted by the user's body which, by reaching deep under the skin, promotes vibrational phenomena of the molecules that alleviate the pain.

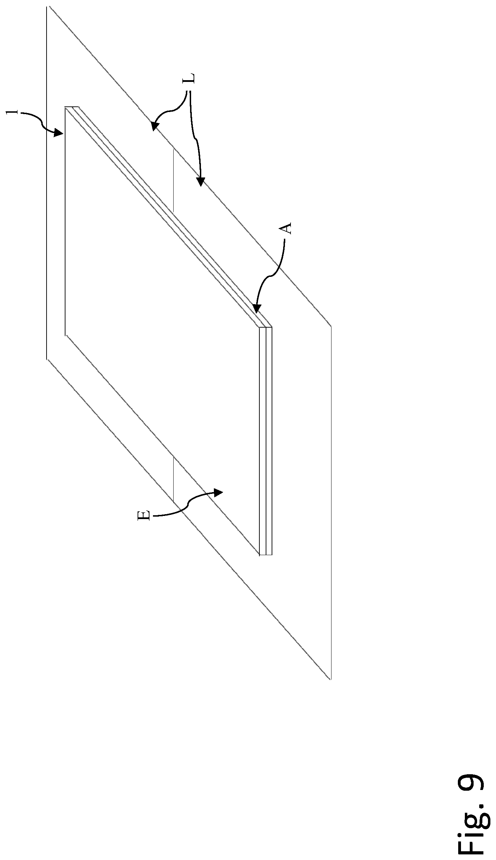

Before focusing on the production process and the line for the production of medical patches 1, with reference to figure 9, it is specified that these have:
- an outer layer E made of polymeric material such as, for example, non-woven polymeric fabric, polyethylene, or polyurethane; and
- an adhesive layer A, applied on a face of the outer layer E and configured to anchor to the user's body, loaded with a chemical compound 4a configured to reflect at least part of the far infrared radiation (FIR) emitted by a body of a user of the medical patch 1.

To the second adhesive layer, two or more protective flaps L are applied side by side and in a removable manner, which cover its face intended to come into direct contact with the user's body.

To apply the medical patch, the user first removes one or more protected flaps L to uncover at least part of the adhesive layer A which can then be brought into contact with the region of their body to be treated. Having done this, if the user has not initially removed all the protective flaps L, they can remove the remaining ones to totally uncover the adhesive layer A and complete the application operation of the patch 1.

To facilitate the user in removing the protective flaps L, these can also project along one or more directions from the shape of the medical patch 1 as shown in figure 9.

That said, the process and then the line for the production of said medical patches 1 will be described below.

This process first of all provides for continuously feeding a laminate 2 having a prevailing extension direction X-X (step A).

This laminate 2 comprises a first layer 3 made of polymeric material, intended to form the outer layer E of the patch 1. The first layer is for example realizable in non-woven polymeric fabric, polyethylene, or polyurethane.

The laminate 2 also comprises a second layer 4, applied on the first layer 3 and made of adhesive material, intended to form the adhesive layer A of the patch 1.

In the second layer 4 is dispersed said chemical compound 4a configured to reflect at least part of the far infrared radiation (FIR) emitted by the user's body.

Preferably, the chemical compound 4a is a compound based on (or substantially composed of) Titanium Dioxide (TiO2), for example, having a granulometry between 1 µm and 20 µm.

According to an aspect, the second layer 4 comprises a polymeric base 4b in which the chemical compound 4a is dispersed, preferably in a homogeneous manner.

The laminate 2 fed in step A) also comprises a protective layer 6, preferably made of siliconized PLA, applied in a removable manner to the second layer 4 on the opposite side with respect to the first layer 3. The protective layer 6 and the first layer 3 are therefore applied on opposite faces of the second layer 4.

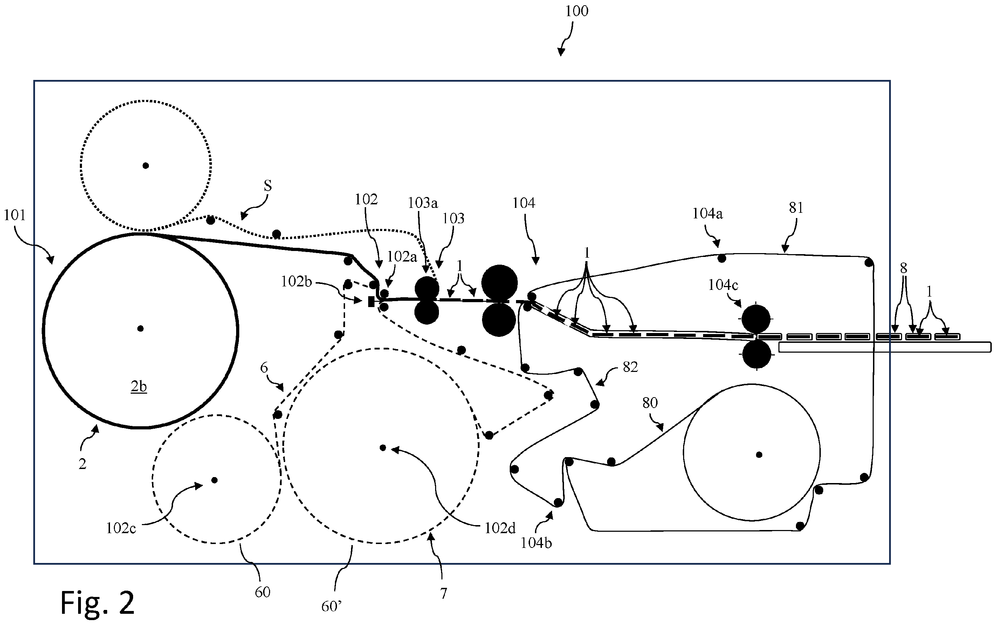

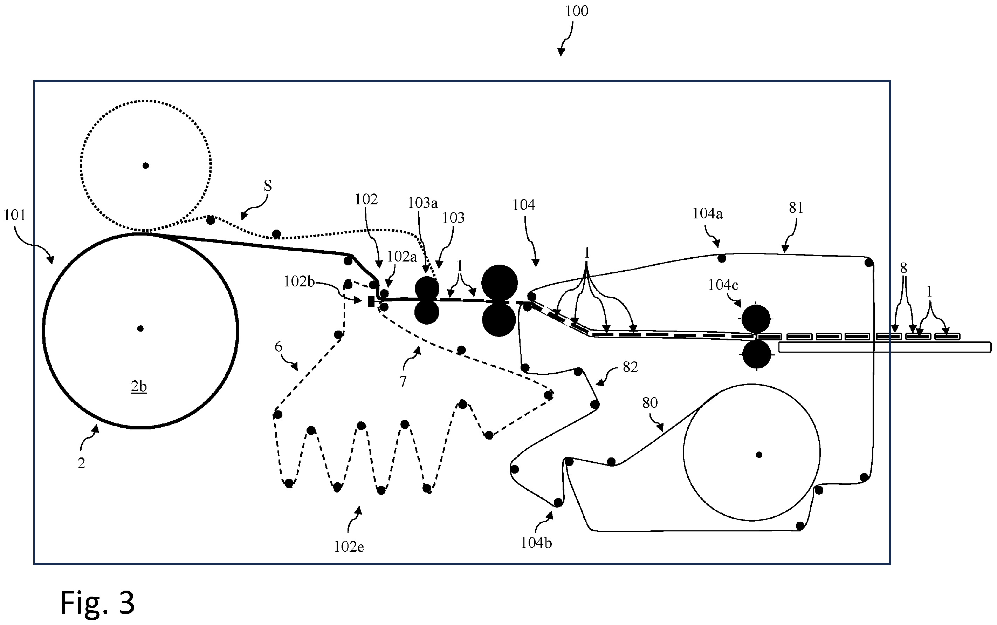

According to an aspect shown in figures 2 and 3, the laminate 2 is supplied on a reel 2b and, therefore, the feeding step A) occurs by gradually unwinding the laminate 2 from the reel 2b.

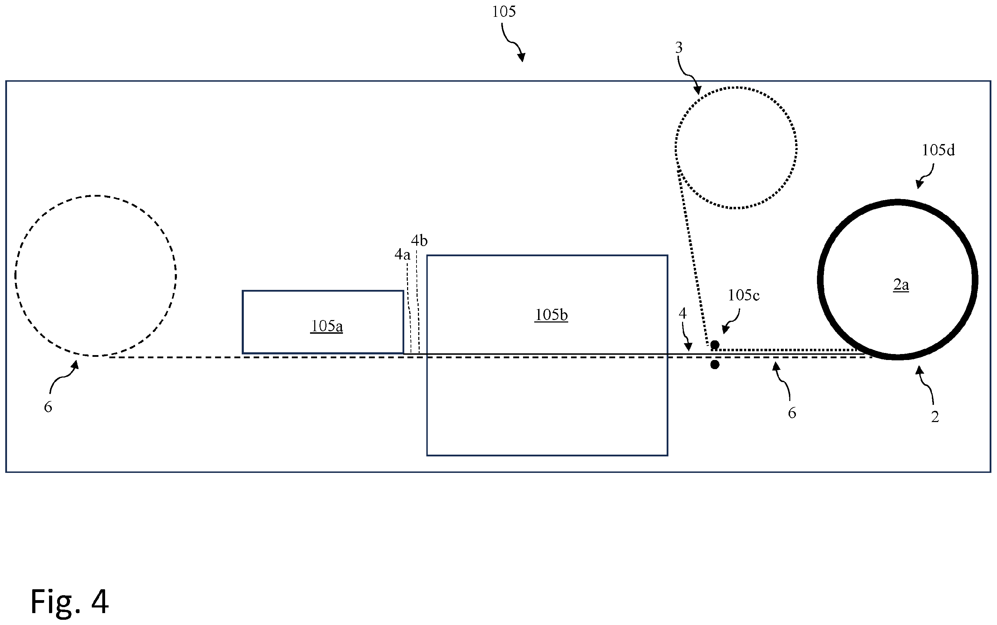

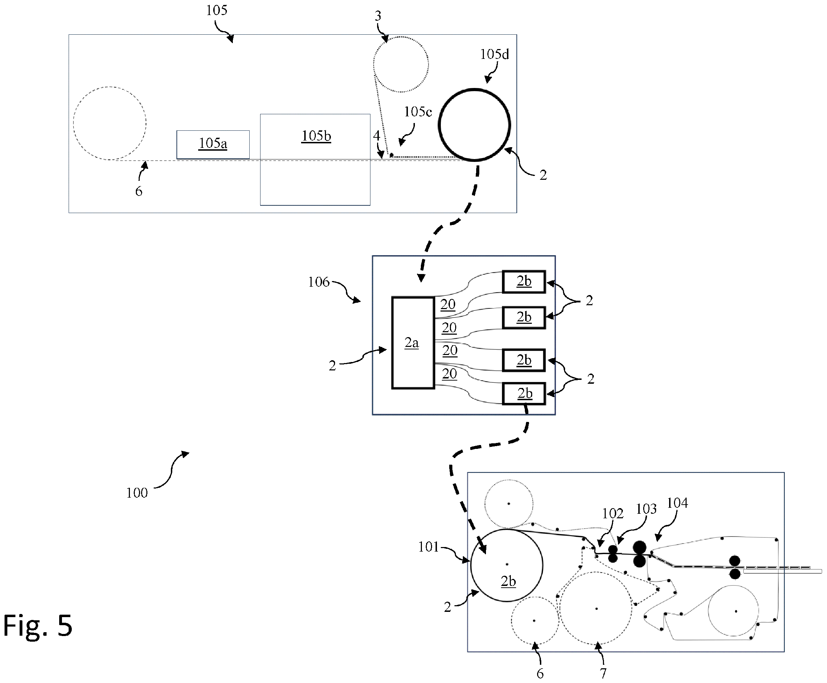

In figures 4 and 5, a possible process that can be used to produce the reel 2b of laminate is schematized.

In detail, in the embodiment shown in figure 4, the process of producing the reel 2b provides for: continuously feeding the protective layer 6 (substep A1), spreading on the protective layer 6 a polymeric matrix 4b in which the chemical compound 4a is dispersed (substep A2), hardening the polymeric matrix 4b by providing heat to produce the second layer 4 on the protective layer 6 (substep A3), laminating the first layer 3 on the second layer 4 on the opposite side with respect to the protective layer 6 (substep A5), and winding the laminate 2 to form a mother reel 2a.

According to an aspect shown in figure 5, the mother reel 2a is then divided into a plurality of reels 2b having a smaller axial extension (substep A6) which are sequentially unreeled to feed the laminate 2.

It is well to specify that by axial extension of a reel is meant the extension of the laminate in the direction of the reel axis. In fact, the axial extension of the reel identifies its width. In light of this, from what has been explained above, it emerges that the width of the mother reel 2a is greater than the width of each reel 2b obtained therefrom. In particular, according to one aspect, the width of the mother reel 2a is equal to the sum of the widths of the reels 2b obtained therefrom.

It is also well to specify that the width of the reels 2b is slightly greater than the prevailing extension of the shape of the patch 1 to be made so as to be able to use the laminate of the reel 2b to produce a plurality of patches 1 by repeatedly die-cutting it along its prevailing extension direction X-X. The mother reel 2b has a width 2b at least double, but preferably equal to 3-7 times, the width of each reel 2b. This allows to obtain two, but preferably 3-7, reels 2b from each mother reel 2a, so as to make the production process of the reels 2b of laminate 1 more efficient.

The plurality of reels 2b is for example obtainable by: unwinding the laminate 2 from the mother reel 2a (substep A6.1), cutting longitudinally - i.e., parallel to its prevailing extension direction X-X - the laminate 2 to obtain a plurality of bands 20 (substep A6.2), and rewinding each band 20 to form a respective reel 2b (substep A6.3).

According to one aspect, the substeps A6.1), A6.2) and A6.3) are performed simultaneously.

As shown in figure 5, the reels 2b obtained in step A6) can then be sequentially used to feed the laminate 2.

Once the laminate 2 has been fed, the production process object of the present description provides for the removal of the protective layer 6, so as to progressively (gradually) expose the second layer 4 along the prevailing extension direction X-X (step B). In this step, the protective layer 6 is progressively delaminated from the second layer 4 to which it is applied.

Step B) preferably occurs in a linear or continuous manner, advancing gradually along the prevailing extension direction X-X of the laminate.

The removal of the protective layer 6 can be performed by means of a mechanical pulling action along a direction transverse to a prevailing extension plane of the laminate 2.

According to one aspect, the protective layer 6 is progressively removed from the second layer 4 during the feeding of the laminate 2.

Downstream of the removal of the protective layer 6, the process provides for applying a removable film 7 to the laminate 2 so as to progressively cover the second layer 4 along the prevailing extension direction X-X (step C).

It is well to specify that the film 7 is applied to the face of the second layer 4 previously uncovered in step B) by removing the protective layer 6. The film 7 is therefore applied on the opposite side with respect to the first layer 3.

The film 7 is for example realizable in siliconized PLA.

In the embodiments shown in figures 2 and 3, once removed, the protective layer 6 is recovered (substep B1) and the film 7 applied in step C) comprises, but is preferably constituted by, the removed protective layer 6.

In detail, in the embodiment shown in figure 2, substep B1) provides for winding the removed protective layer 6 so as to form a recovery reel 60 which is subsequently usable to feed the film 7, should the latter comprise or be constituted by the removed protective layer 6.

Differently from the embodiment of figure 2, in the embodiment of figure 3 the protective layer 6 removed in step B) is reapplied to the second layer 4 in the form of film 7 in step C). In the embodiment of figure 2, the removed protective layer 6 is therefore directly fed as film 7.

If on the one hand the embodiment of figure 3 is advantageous in that it does not require removal/installation operations of the recovery reel 60, on the other hand the embodiment of figure 2 allows to apply films 7 made of a material different from the removed protective layer 6 or to treat/modify the protective layer 6 before reapplying it in the form of film 7.

It is also worth noting that the embodiment of figure 2, differently from the embodiment of figure 3, by decoupling the operations of removing the protective layer 6 from those of applying the film 7, is not subject to problems of flow synchronization.

In more detail, step C) of applying the film provides for: continuously feeding the film 7 (substep C1), cutting the fed film 7 so as to divide it into two or more strips 70 that extend predominantly along a respective longitudinal direction L-L (substep C2), and applying the strips 70 on the second layer 4 so that they are side by side (not overlapping) and with their respective longitudinal directions L-L oriented parallel to the prevailing extension direction X-X of the laminate 2 (substep C3).

According to one aspect, in substep C2, the film 7 is progressively cut during its feeding so as to be progressively partitioned into the two or more strips 70 which are progressively applied to the second layer 4 of the laminate 2 in substep C3.

According to a further aspect, the strips 70 are made by cutting the film 7 parallel to the direction along which it predominantly extends and, therefore, have a constant width.

In a preferred embodiment, the strips 70 are applied to the second layer 4 of the laminate 2 immediately after being obtained by cutting the film 7. That is to say that it is preferable that step C3) is carried out immediately downstream of step C2). In other words, in accordance with what is shown in the embodiment of figure 5, the strips 70 are cut in the vicinity of the area in which they are applied to the second layer 4 of the laminate 2.

Advantageously, this allows for greater precision in the positioning of the strips 70 in a side-by-side position.

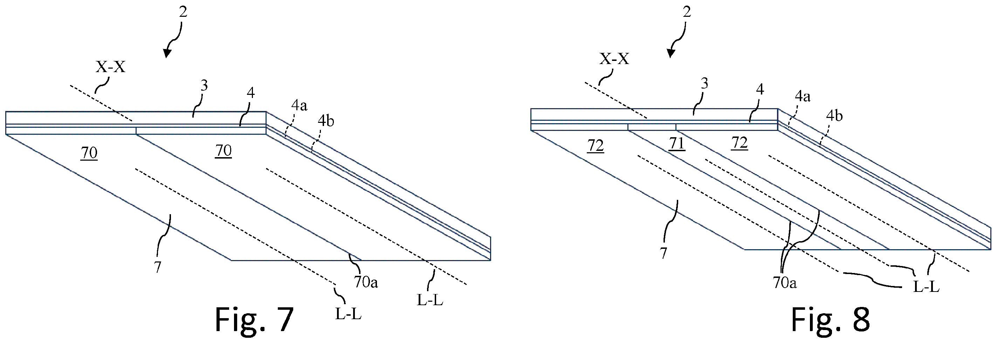

In figures 7 and 8, examples of the output from step C3 are reported.

In accordance with what is shown in the embodiments shown in figures 7 and 8, each strip 70 obtained from the film 7 is delimited transversely to the respective longitudinal direction L-L by opposite longitudinal edges 70a. When applied to the second layer 4 of the laminate 2, the strips 70 have their respective longitudinal edges extending parallel and in contact, i.e., in contact for their entire extension.

Furthermore, always with reference to the embodiments shown in figures 7 and 8, downstream of step C3), the strips 70 cover the second layer 4 seamlessly transversely to the prevailing extension direction X-X. That is to say that, when applied to the second layer 4 of the laminate 2, the strips 70 are placed side by side so as to be neither spaced apart nor overlapping.

According to an embodiment, in substep C2), the film 8 is cut so as to be simultaneously divided into a central strip 71 and two side strips 72. In substep C3), the side strips 72 are applied to the second layer 4 of the laminate 2 sideby side from opposite sides to the central strip 71, in accordance with what is shown in figure 8.

Downstream of the application of the film in step C), the production process provides for repeatedly die-cutting the laminate 2 so as to cut the first and second layers 3, 4 in a plurality of regions spaced apart along the prevailing extension direction of the laminate 2 (step D). By so doing, a plurality of patches 1 are obtained, arranged in succession on the film 7 along the prevailing extension direction X-X.

According to one aspect, in the die-cutting step the film 7 is not cut, i.e., only the first and second layers 3, 4 (and any other layers of the laminate) are cut. This means that, downstream of the die-cutting process, the plurality of patches 1 are jointly transportable by moving the film 7 which therefore acts as a means of transport.

It is well to specify that, as is customary in die-cutting operations, downstream of the die-cutting a web (scrap or waste matrix) S is removed from the film 7, leaving only the patches 1 on it. In the embodiments shown in figures 2 and 3, after being removed, the web S is wound to form a web reel.

This done, the production process provides for cutting the film 7 so as to separate the patches 1 (step E) and then enclosing each patch 1 in a respective casing or sachet 8 (step F).

According to one aspect, step F) provides for: the continuous feeding of a first packaging film 81 and a second packaging film 82 for packaging from opposite parts/sides with respect to the patches output from the die-cutting (substep F1); and the connection of the first film 81 to the second film 82, so as to enclose each patch 1 inside the respective casing 8 (substep F2).

The fed first and second films 81, 82 are therefore intended to form two opposite faces of the casings 8.

The first and second films 81, 82 can for example be obtained by longitudinally dividing a sheet 80 made of packaging material so as to partition it into the first and second films 81, 82.

The connection between the first and second films 81, 82 can be made by means of known techniques of gluing or heat-sealing the first film 81 to the second film 82.

In an embodiment, the patches 1 separated in step E are individually enclosed in respective casings 8 by connecting the first film 81 to the second film 82 along a connection edge (not shown in the figures) which surrounds, preferably in a close manner, the shape of each patch 1. The connection of the first film 81 to the second film 82 along the connection edge leads to the creation of a closed pocket around each patch 1.

In the embodiments shown in figures 2 and 3, the first and second films 81, 82 fed continuously from opposite sides of the patches are used to transport the patches 1 downstream of their separation in step E), until their enclosure in the respective casings. In other words, according to one aspect, the first and second films 81, 82 are used as means of transport for the patches 1 during at least part of the packaging process.

The present description also relates to a line 100 for the production of medical patches 1 for the treatment of pain by means of heat, preferably by implementing the production process described above.

Said line 100 comprises a feeding station 101 for the continuous feeding of the laminate 2.

In the embodiments shown in figures 2 and 3, the feeding station 101 comprises an unwinding unit configured to progressively unreel a reel 2b of laminate 2.

The fed laminate 2 then follows a specific path along which various processing stations are arranged, adapted to act in succession on the laminate to transform it into the plurality of patches 1 enclosed in their respective casings. To this end, the line 100 comprises a movement system, for example with rollers, adapted to convey the laminate 2 or its components.

As regards the characteristics of the fed laminate 2, reference is made to what is explained above.

Downstream of the feeding station 101, the line 100 comprises a replacement station 102 configured to receive the laminate 2 and replace its protective layer 6 with the removable film 7 described above. The replacement station 102 is therefore configured to implement steps B) and C) of the production process described above.

In detail, the replacement station 102 is configured to progressively remove the protective layer 6 of the laminate 2 and then progressively apply in its place the two or more strips 70 of film 7 side by side in accordance with what is reported above.

The replacement station 102 comprises cutting members 102b adapted to divide the film 7 into the two or more strips 70 of film 7 which extend predominantly along respective longitudinal directions L-L.

The replacement station 102 also comprises a pair of opposing rollers 102a, arranged downstream of the cutting members 102b, configured to laminate the film 7 on the second layer 4 after the removal of the protective layer 6. In particular, the opposing rollers 102a are configured to apply the strips 70 obtained from the cutting of the film 7 on the second layer 4 of the laminate 2 in a side-by-side manner and with the respective longitudinal directions L-L oriented parallel to the prevailing extension direction of the laminate 2. For more details on the arrangement of the strips 70 on the second layer 4 of the laminate 2, reference is made to what is described above.

According to an aspect shown in figures 2 and 3, the cutting members 102b are arranged in proximity to the pair of opposing rollers 102a. Advantageously, this allows to increase the positioning precision of the strips 70 of film 7 on the second layer 4 of the laminate 2, thus preventing the formation of undesired overlaps.

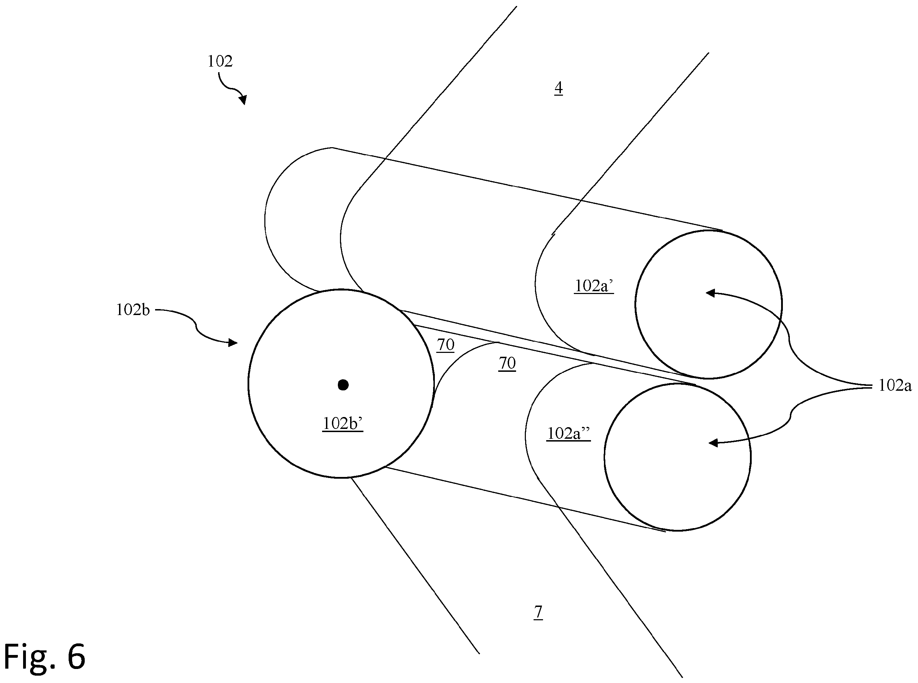

In the embodiment of figure 6, the pair of opposing rollers 102a comprises: a first roller 102a' configured to act on the first layer 3 to bring the second layer 4 into contact with the strips 70 of the film 7; and a second roller 102a", opposite the first roller 102a', configured to act on the strips 70 of the film 7 to bring them into contact with the second layer 4 of the laminate 2. In this embodiment, the cutting members 102b are arranged in proximity to the second roller 102a" so as to divide the film 7 into strips 70 just before their application on the second layer 4 of the laminate 2.

According to one aspect, in the embodiment of figure 6, the cutting members 102b are operatively associated with the second roller 102a" so as to cut the film that runs on the second roller 102a".

The cutting members 102b comprise for example a discoidal cutting element 102b' having a cutting edge and configured to be opposite the second roller 102a".

According to one aspect, the second roller 102a" offers a reaction to the cutting action applied by the cutting members 102b.

It is well to specify that, if the division of the film into three or more strips is provided, the cutting members 102b can comprise two or more side-by-side cutting elements and each functionally associated with a respective portion of the second roller 102a".

The replacement station 102 can be configured to recover the removed protective layer 6 and, in particular, reuse it as at least part of the film 7. Two possible solutions for recovering the removed protective layer 6 are shown in figures 2 and 3.

In the embodiment of figure 2, the replacement station 102 is provided with winding members 102c configured to wind the removed protective layer 6 so as to form a recovery reel 60, and unwinding members 102d configured to unwind a previous recovery reel 60' previously formed by the winding members 102c. The unwinding members 102d therefore allow to feed the previously removed protective layer 6 of which the previous recovery reel 60' is made as film 7 to the cutting members 102b and to the pair of opposing rollers 102.

In the embodiment of figure 3, the replacement station 102 comprises recovery members 102e configured to receive the removed protective layer 6 and feed it to the cutting members 102b and to the pair of opposing rollers 102.

Downstream of the replacement station 102, the line 100 comprises a die-cutting station 103 configured to repeatedly die-cut the laminate 2 so as to cut the first layer 3 and the second layer 4 in a plurality of its regions along the prevailing extension direction X-X, so as to obtain a plurality of patches 1 arranged in succession on the film 7 along the same prevailing extension direction X-X. The die-cutting station 103 is therefore configured to perform step D) of the process described above.

According to one aspect, the die-cutting station 103 comprises a die 103a configured to cut the first and second layers of the laminate 2 and not the strips 70 of the film 7. Therefore, preferably, the die-cutting station 103 is configured to cut only the first and second layers 3, 4 of the laminate 2. It is worth noting that the fact that the die-cutting does not cut (at least completely) also the strips 70 of film 7 is particularly advantageous as it allows the latter to be used as a means of transport for the patches 1.

The die-cutting station 103 also comprises web removal members 103a configured to remove the web S from the film 7 downstream of the die-cutting operation. Once the web S is removed from the strips 70 of the film 7, the patches 1 arranged in succession will therefore remain.

The line 100 comprises, downstream of the die-cutting station 103, a packaging station 104 configured to cut the film 7 so as to separate the plurality of patches 1 and enclose each patch 1 in a respective casing 8. The packaging station 104 is therefore configured to perform steps D) and E) of the production process described above.

To separate the patches 1, i.e., to perform step D) of the production process, the packaging station 104 comprises separation members configured to cut the strips 70 of film 7 transversely to the longitudinal directions L-L between two successive patches 1.

In the embodiments shown in figures 2 and 3, the packaging station 104 comprises first and second feeding members 104a and 104b, arranged downstream of the die-cutting station 103, configured to respectively feed the first and second films 81, 82 for packaging from opposite sides of the patches 1 output from the die-cutting station 103.

In the embodiments shown in figures 2 and 3, the packaging station 104 also comprises joining members 104c configured to connect the first and second films 81, 82 for packaging so as to enclose each patch in its respective casing 8.

According to one aspect, the joining members 104c are configured to connect the first and second films 81, 82 along a connection edge (not shown in the figures) surrounding the shape of each patch 1. The connection of the first film 81 to the second film 82 along the connection edge leads to the creation of a closed pocket around each patch 1.

In the embodiment of figure 5, the line 100 comprises, upstream of the feeding station 101, a laminate formation station 105 adapted to produce the laminate 2.

In detail, with reference to figure 4, said laminate formation station 105 comprises spreading members 105a configured to spread on the protective layer 6 a polymeric matrix 4b in which is dispersed the chemical compound 4a configured to reflect at least part of the far infrared radiation emitted by a body of a user of the medical patch 1.

The spreading members 105a are therefore configured to perform step A2 described above on the protective layer 6 fed, for example, by unwinding it from a reel on which it is wound.

The laminate formation station 105 also comprises hardening members 105b, arranged downstream of the spreading members 105a, configured to harden the polymeric matrix 4b to form a second adhesive layer 4 on the protective layer 6.

According to one aspect, the hardening members 105b are configured to provide heat to the polymeric matrix 4b to promote the hardening process. In particular, the hardening members 105b comprise for example an oven having a temperature-controlled chamber traversable by the protective layer 6 on which the polymeric matrix 4b is spread.

The hardening members 105b are therefore configured to perform step A3 described above.

The laminate formation station 105 also comprises lamination members 105c configured to laminate the first layer 3 on the second layer 4 on the opposite side with respect to the protective layer 6, so as to form the laminate 2 described above.

According to one aspect, the lamination members 105c comprise one or more lamination rollers configured to receive the first layer 3, for example from a respective reel, and apply it on the second layer 4 by exerting a force directed transversely to the prevailing extension plane of the second layer 4.

The lamination members 105c are therefore configured to perform step A4 described above.

The laminate formation station 105 may also comprise winding members 105d configured to wind the laminate 2 on a mother reel 2a and, therefore, perform step A5 described above.

In the embodiment of figure 5, the line 100 may also comprise a cutting-rewinding station 106 configured to divide the mother reel 2a into a plurality of reels 2b having a smaller axial extension than the mother reel 2a.

The cutting-rewinding station 106 is therefore configured to perform step A6) and, in particular, its substeps A6.1) - A6.3).

The reels 2b of laminate 2 thus obtained are then sequentially mounted in the feeding station 101 which, by unwinding them one after the other, feeds the laminate 2 to the subsequent processing stations.

Obviously, a person skilled in the art may make numerous equivalent modifications to the variants described above, without thereby departing from the scope of protection defined by the attached claims.

## Claims

1. Process for the production of medical patches (1) for treating pain by means of heat, wherein:
- the process comprises the following steps:
A) continuously feeding a laminate (2) having a prevailing extension direction (X-X) and comprising:
- a first layer (3) made of polymeric material,
- a second layer (4) applied onto the first layer (3) and made of adhesive material, in the second layer (4) being dispersed a chemical compound (4a) configured to reflect at least part of the far infrared radiation (FIR) emitted by a body of a user of the medical patch (1),
- a protective layer (6) removably applied to the second layer (4) on the opposite side with respect to the first layer (3),
B) removing the protective layer (6) from the fed laminate (2) so as to progressively uncover the second layer (4) along the prevailing extension direction (X-X),
C) applying a removable film (7) to the laminate (2) so as to progressively cover the second layer (4) along the prevailing extension direction (X-X),
D) repeatedly die-cutting the laminate (2) so as to cut the first layer (3) and the second layer (4) in a plurality of regions spaced apart along the prevailing extension direction (X-X) and obtaining a plurality of patches (1) arranged in succession on the film (7) along the prevailing extension direction (X-X),
E) cutting the film (7) so as to separate the patches (1),
F) enclosing each patch (1) in a respective casing (8),
**characterized in that** step C) of applying the film (7) comprises the substeps of:
C1) continuously feeding the film (7),
C2) cutting the fed film (7) in order to divide it into two or more strips (70) extending predominantly along a respective longitudinal direction (L-L), C3) applying the strips (70) on the second layer (4) side by side with their respective longitudinal directions (L-L) oriented parallel to the prevailing extension direction (X-X) of the laminate (2).

2. Process according to claim 1, wherein in substep C3) the strips are applied to the second layer (4) immediately after the cutting substep C2).

3. Process according to any one of the preceding claims, wherein:
- the step B) comprises a substep B1) wherein the removed protective layer (6) is recovered,
- the film (7) applied in step C) comprises the protective layer (6) recovered in the substep B1).

4. Process according to claim 3, wherein:
- the substep B1) provides for winding the removed protective layer (6) so as to form a recovery reel (60),
- the substep C1) provides for unwinding the recovery reel (60) obtained from substep B1).

5. Process according to claim 3, wherein the removed protective layer (6) is directly fed as film (7) in substep C1).

6. Process according to any one of the preceding claims, wherein step F) provides for the substeps of:
F1) continuously feeding a first packaging film (81) and a second packaging film (82) from opposite sides of the patches (1) output from the die-cutting step D), the first packaging film (81) and the second packaging film (82) being intended to make opposite faces of the casings (8) enclosing the patches (1),
F2) after separating the patches in step E), connecting the first packaging film (81) to the second packaging film (82) so as to enclose each patch (1) in the respective casing (8).

7. Process according to any one of the preceding claims, wherein in the die-cutting step D) the film (7) is not cut.

8. Process according to any one of the preceding claims, wherein each strip (70) obtained from the film (7) is delimited transversely to the longitudinal direction (L-L) by opposite longitudinal edges (70a), in the substep C3) the strips (70) are applied to the second layer (4) of the laminate (2) with the respective longitudinal edges (70a) extending parallel and in contact.

9. Process according to any one claim, wherein when applied in the substep C3) the strips (70) coat the second layer (4) seamlessly transversely to the prevailing extension direction (X-X).

10. Process according to any one of the preceding claims, wherein:
- in the substep C2) the film (7) is cut so as to be simultaneously divided into a central strip (71) and two side strips (72),
- in the substep C3) the side strips (72) are applied to the second layer (4) of the laminate (2) side by side from opposite sides to the central strip (71).

11. Line (100) for the production of medical patches (1) for treating pain by means of heat, said line (100) comprising:
- a feeding station (101) configured to continuously feed a laminate (2) having a prevailing extension direction (X-X) and comprising:
- a first layer (3) made of polymeric material,
- a second layer (4) applied onto the first layer (2) and made of adhesive material, in the second layer (4) being dispersed a chemical compound (4a) configured to reflect at least part of the far infrared radiation (FIR) emitted by a body of a user of the medical patch (1),
- a protective layer (6) removably applied to the second layer (4) on the opposite side with respect to the first layer (3),
- a replacement station (102) configured to receive the laminate (2) from the feeding station (101) and replace the protective layer (6) with a removable film (7),
- a die-cutting station (103) configured to repeatedly die-cut the laminate (2) so as to cut the first layer (3) and the second layer (4) in a plurality of regions along the prevailing extension direction (X-X) so as to obtain a plurality of patches (1) arranged in succession on the film (7) along the prevailing extension direction (X-X),
- a packaging station (104) configured to cut the film (7) so as to separate the plurality of patches (1) and to enclose each patch (1) in a respective casing (8),
**characterized in that** the replacement station (102) comprises:
- a pair of opposing rollers (102a) configured to laminate the film (7) on the second layer (4) after removal of the protective layer (6),
- cutting members (102b) configured to divide the film (7) into two or more strips (70) extending predominantly along a respective longitudinal direction (L-L), the cutting members (102b) being arranged upstream of the pair of opposing rollers (102a), the pair of opposing rollers (102a) being configured to apply the strips (70) on the second layer (4) side by side and with the respective longitudinal directions (L-L) oriented parallel to the prevailing extension direction (X-X) of the laminate (2).

12. Line (100) according to claim 11, wherein the cutting members (102b) are arranged in proximity to the pair of opposing rollers (102a).

13. Line (100) according to claim 12, wherein:
- the pair of opposing rollers (102a) comprises:
- a first roller (102a') configured to act on the first layer (3) to bring the second layer (4) into contact with the strips (70) of the film (7),
- a second roller (102a"), opposite the first roller (102a'), configured to act on the strips (70) of the film (7) to bring said strips (70) into contact with the second layer (4) of the laminate (2)
- the cutting members (102b) are arranged in the vicinity of the second roller (102a") so as to divide the film (7) into strips (70) just before said strips (7) come into contact with the second layer (4) of the laminate (2).

14. Line (100) according to any one of the claims from 11 to 13, wherein:
- the film (7) comprises the removed protective layer (6),
- the replacement station (102) is provided with:
- winding members (102c) configured to wind the protective layer (6) removed so as to form a recovery reel (60),
- unwinding members (102d) configured to unwind a previous recovery reel (60') previously formed by the winding members (102c) and to feed the removed protective layer (6) as film (7) to the cutting members (102b) and then to the pair of opposing rollers (102a).

15. Line (100) according to any one of the claims from 11 to 14, wherein the die-cutting station (103) comprises at least one die (103a) configured to cut the first layer (3) and the second layer (4) of the laminate (2) and not the strips (70) of the film (7).
